# FASCICULE DE BREVET EUROPEEN

(11) **EP 0 509 904 B1**
(45) Date de publication et mention de la délivrance du brevet: **28.02.1996**
(21) Numéro de dépôt: 92401043.2
(22) Date de dépôt: 14.04.1992
(51) Int. Cl.: A61K 7/42, B01J 13/20

(54) **Micro-capsules de filtres solaires, leur procédé de préparation, les compositions cosmétiques et pharmaceutiques les comprenant et leurs applications**
Mikroverkapseltes Sonnenschutzmittel, das Verfahren zur Herstellung, kosmetische und pharmazeutische Zubereitungen mit Mikrokapseln und Verwendung derselben
Microencapsulation of sunscreen products, their preparation process, cosmetic and pharmaceutical compositions containing said microcapsules and their applications

(30) Priorité: 19.04.1991 FR 9104881
(43) Date de publication de la demande: 21.10.1992
(73) Titulaire: ROUSSEL UCLAF, F-93230 Romainville (FR)
(72) Inventeur: Lahmani, Paul, F-37000 Tours (FR); Simoneau, Lise, F-13010 Marseille (FR)
(74) Mandataire: Jacobi, Markus Alexander

(56) Documents cités:
- US-A- 3 872 024
- SOAP, PERFUMERY AND COSMETICS, vol. 50, no. 12, décembre 1977, pages 501-503, United Trade Press Ltd., GB; D. GREFF: "Microencapsulation of cosmetic products"

## Description

Depuis longtemps l'industrie cosmétique met à la disposition des personnes, des produits tels des gels, des crèmes, des huiles incorporant des filtres solaires. Ce sont des substances chimiques qui absorbent les rayonnements UVA et/ou B.

Il est en effet intéressant de limiter les effets du rayonnement solaire au niveau cutané, en particulier ceux des UVB et A. Les premiers provoquent l'érythème solaire et à plus long terme des altérations cutanées bénignes et malignes ; les seconds sont le principal facteur du vieillissement cutané.

Les produits protecteurs solaires sont d'une très grande utilité car ils apportent réellement une protection de la peau qui est mesurée par ce que l'on appelle un facteur de protection.

Cependant leur présence sur la peau, si elle est très bénéfique par la protection contre les radiations UVA et B, n'est pas complètement inoffensive. A tel point que la plupart des législations ont constitué des listes positives de filtres solaires ; ce qui revient à dire que les produits cosmétiques ne peuvent contenir que les filtres inscrits sur cette liste et qui ont fait la preuve d'une certaine innocuité. Tel est le cas en France et dans la CEE. Aux USA, les produits contenant des filtres solaires sont considérés comme des quasi-médicaments et font partie d'une catégorie à part ; les produits O.T.C. (Over The Counter).

Il apparaissait donc judicieux de trouver un moyen de mettre des filtres solaires sur la peau sans qu'ils soient en contact direct avec celle-ci.

L'un des moyens utilisés est l'encapsulation ou microencapsulation. Ainsi l'art antérieur décrit des microcapsules notamment dans les brevets USP 4,904,524 et EP 0,254,447 mais à la différence des microcapsules, objet de la présente invention, les microcapsules décrites dans l'art antérieur qui peuvent renfermer des filtres solaires comme dans US 4,904,524 ne sont pas étanches. En effet, il faut que les micro-capsules soient dures pour ne pas être écrasées lors de l'application, résistantes pour ne pas se laisser ramollir en étant en contact avec les autres composants de la composition cosmétique, et non poreuses pour ne pas laisser fuir les filtres solaires à l'extérieur.

La présente invention a ainsi pour objet des micro-capsules comprenant un ou plusieurs filtres solaires caractérisées en ce que lesdites microcapsules sont durcies de façon à être étanches.

Dans la présente invention, les micro-capsules contenant le ou les filtres solaires sont durcies de façon à être étanches pour ne pas laisser échapper le ou les filtres solaires lors de la fabrication, de la conservation ou de l'étalement sur la peau.
La présente invention a aussi particulièrement pour objet les microcapsules telles que définies ci-dessus caractérisées en ce que la paroi des microcapsules comprend de la gélatine durcie de préférence par de la glutaraldéhyde. De plus, la taille des micro-capsules de l'invention doit être petite pour que celles-ci ne soient pas senties sur la peau à l'étalement et pour assurer une homogénéité de présence en filtre au-dessus de la peau. Dans US 3,872,024 et dans Soap. Parfumery and Cosmetics vol. 50, N° 12, 1977, p. 501-503 des microcapsules sont décrites, mais n'ayant pas une taille très spécifique. La présente invention a pour objet les microcapsules telles que définies ci-dessus caractérisées en ce que la taille des microcapsules est comprise entre 30 et 80 micromètres.

La présente invention a plus particulièrement pour objet les microcapsules telles que définies ci-dessus caractérisées en ce que la taille des microcapsules est comprise entre 40 et 45 micromètres.

La présente invention a encore plus précisément pour objet les microcapsules telles que définies ci-dessus caractérisées en ce que le ou les filtres solaires que lesdites microcapsules comprennent sont purs ou dilués dans une huile et choisis notamment dans la liste suivante : octyl méthoxycinnamate et notamment le 4-méthoxycinnamate d'éthyl hexyle, méthylbenzylidène camphre, isoamyl méthoxycinnamate, octydiméthyl paba, paba ou acide 4-amino benzoïque, homosalate, octyl salicylate, butyl méthoxydibenzoylméthane, isopropyl dibenzoylméthane, oxybenzone, octyl triazone.

Cette liste n'est donnée qu'à titre indicatif, tout filtre solaire ou toute substance chimique à effet filtrant liposoluble pouvant être utilisé.

Les micro-capsules pouvant contenir une phase grasse constituée de 50 à 100 % de filtres solaires et d'une autre huile couramment utilisée en cosmétologie, cette huile peut, par exemple être choisie parmi les produits suivants : huile de vaseline, perhydrosqualène, polyisobutène, dioctylcyclohexanne, huile de tournesol, huile de ricin, huile d'onagre, huile d'olives, huile de jojoba, huile de rosier muscat, huile de sésame, dioctonoate de propylène glycol, dicaprate/dicaprylate de propylène glycol, isocétyl isostéarate, polydiméthyl cyclosiloxame, octyl dodécanol, polyphényl méthylsiloxanne, huile de lanoline.

La pharmacopée donne la définition suivante de la microencapsulation :
"Les micro-capsules sont des produits solides constitués d'une enveloppe elle-même solide contenant un liquide, un solide ou une substance pâteuse. Elles sont obtenues par divers procédés, tels que la coacervation, la polymérisation inter-faciale.

Les différents procédés de microencapsulation fournissent des microparticules de structure et de morphologie très variées. Une classification simple tente de regrouper ces différentes structures en deux catégories :
- les microsphères ou système matriciel, qui sont des particules sphériques pleines dans lesquelles le principe actif à encapsuler est dispersé de façon homogène dans le matériau support (réseau continu de matériau support) ;
- les micro-capsules ou système réservoir, qui sont des particules sphériques creuses, le principe actif se trouvant au coeur, à l'état solide, liquide ou pâteux, entouré par une membrane solide dénuée de principe actif. C'est cette catégorie qui est utilisée dans la présente invention. Des exemples de préparations de micro-capsules sont également décrits dans les brevets européens EP 0.025.379 et EP 0.399.911.

Le procédé préférentiellement utilisé dans le cadré de la présente invention est un procédé de coacervation complexe.

La présente invention a également pour objet un procédé de préparation de microcapsules telles que définies ci-dessus comprenant une préparation de solution colloïdale et une dispersion de la substance à encapsuler dans cette solution, une séparation de phase (ou coacervation) et formation d'un système ternaire par variation du pH et une encapsulation de la substance dispersée, caractérisé en ce que ce procédé comprend ensuite une phase de durcissement de la paroi desdites microcapsules et séparation desdites microcapsules afin d'obtenir des particules solides.

Les valeurs de pH utilisées sont comprises entre 3 et 7 et de préférence entre 4 et 5.

Le terme de coacervation (du latin acervus) signifie agrégation. Il décrit le phénomène de désolvatation de macromolécules (agrégation de macromolécules entre elles) conduisant à une séparation de phase au sein de solutions colloidales initialement homogènes et diluées.

En effet, une substance macromoléculaire en présence d'un solvant donne une solution colloidale (les molécules d'eau sont fixées par les macromolécules).

Sous l'effet de la variation d'un paramètre physico-chimique, le pH, les forces d'attractions entre les molécules sont plus fortes que celles qui les lient au solvant (il y a donc désolvatation), et une nouvelle phase apparaît -le coacervat-, constitués par des gouttelettes individualisées riches en substances macromoléculaires, qui vont envelopper les particules à la manière d'une capsule.

La coacervation complexe résulte de l'interaction de deux polymères de charge électrique opposée (dont au moins un des deux doit être un colloide) comme par exemple : gélatine et gomme arabique, gélatine et alginate de sodium, gélatine et polyphosphate de sodium.

Cette interaction entre les deux polymères peut aboutir à un complexe dont la solubilité est réduite à un tel point qu'une séparation de phase intervient -séparation en deux phases dont l'une est pauvre en polymères et riche en solvant, et l'autre riche en polymères (P+P-).

La gélatine est une macroprotéine. La coacervation complexe fait intervenir les groupements ionisés de celle-ci. Par exemple, à pH inférieur au point isoélectrique, la gélatine est chargée positivement.

Ainsi en ajustant le pH du milieu (car les charges sur la chaîne varient en fonction du pH) où se trouvent les deux polymères, les charges positives de la gélatine (unité lysine) peuvent être exactement neutralisées par les charges négatives du deuxième polymère (gomme arabique, polyphosphate de sodium qui est un polymère non organique).

Dans des conditions préférentielles de mise en oeuvre de l'invention, le procédé est réalisé de la façon suivante.

La coacervation complexe peut se dérouler en quatre phases :
- préparation de la solution colloidale, dispersion de la substance à encapsuler dans cette solution,
- séparation de phase (ou coacervation) et formation d'un système ternaire par variation du pH,
- encapsulation de la substance dispersée,
- durcissement de la paroi et séparation des micro-capsules afin d'obtenir des particules solides.

Le 1er polymère est la gélatine qui est constituée d'amino-acides.

La liaison entre monomères s'effectue par liaison peptidique. La structure fine de la gélatine peut être définie comme une séquence de type (glycine-X-Y)n, les acides-aminés étant souvent, respectivement, de la proline et de l'hydroxyproline.

Le 2ème polymère est le polyphosphate de sodium, préférentiellement l'hexamétaphosphate de sodium. L'agent durcisseur est préférentiellement le glutaraldéhyde, en solution à 25 % dans l'eau. La phase huile est constituée d'un filtre solaire, par exemple le 4-méthoxycinnamate d'éthyl hexyle ou octyl méthoxycinnamate (PARSOL MCX®).

Des exemples de réalisation de ce procédé figurent ci-après dans la partie expérimentale.

Le procédé initial tel que nous venons de le décrire peut faire l'objet de certaines variations, et lors de la mise au point de la présente invention l'adjonction de tensio-actifs, la vitesse d'agitation, la forme des pales d'agitation, ont été prises en compte.

La modification la plus intéressante du procédé initial consiste en l'adjonction lors de la formation de l'émulsion primitive de tensio-actifs non ioniques à la dose de 1 à 5 %.

De même, la vitesse d'agitation peut être choisie entre 300 et 1500 tours/mn et de préférence de 800 à 1000 tours/mn.

En fonction des conditions choisies, le rendement de la micro-encapsulation, ainsi que la taille des micro-capsules, seront variables.

La présente invention nous permet, en fonction du procédé choisi, de fabriquer des micro-capsules dont la taille varie entre 30 microns et 150 microns.

Le procédé initial modifié par l'adjonction de tensioactif non ionique, permet d'obtenir une population de taille allant de 30 à 80 microns, avec un maximum homogène entre 40 et 45 microns.

Ce tensio-actif peut être choisi, par exemple, dans la liste suivante non exhaustive : MONTANE 20, MONTANE 80, (Monolaurate de Sorbitan POE), LAURYL SULFATE de Sodium, TWEEN 20, TWEEN 80, (Polysorbate 20 ou 80), DEHYTON K, (cocomidopronybetaine).

De préférence, le tensio-actif utilisé sera non ionique et par exemple le MONTANE 20 (monolaurate de Sorbitan POE).

La présente invention a ainsi pour objet le procédé tel que défini ci-dessus caractérisé en ce que l'on ajoute un tensio-actif non ionique tel que le MONTANE 20 dans la dispersion de la substance à encapsuler.

La quantité de tensio-actif utilisé peut être de 0,5 % à 5 % de la préparation et de préférence 1 %.

Les micro-capsules obtenues en encapsulant un ou plusieurs filtres tels que ceux ci-dessus, par l'un des procédés décrits précédemment, peuvent être utilisées dans une composition cosmétique destinée à protéger la peau humaine des rayons UVA et UVB.

La présente invention a ainsi particulièrement pour objet des compositions cosmétiques caractérisées en ce qu'elles comprennent des microcapsules telles que définies ci-dessus et tout particulièrement des compositions cosmétiques anti-solaires comprenant l'incorporation dans un produit cosmétique de filtres solaires micro-encapsulés, de telle sorte qu'il n'y ait aucun contact entre les filtres solaires et la peau, ces compositions cosmétiques pouvant également contenir des réflecteurs physiques de rayons UVA et UVB.

Ces compositions cosmétiques peuvent être :
- des gels aqueux,
- des gels gras,
- des émulsions simples, eau dans huile,
- des émulsions simples, huile dans eau,
- des émulsions multiples,
- eau dans huile dans eau ou huile dans eau dans huile, par exemple :
- une émulsion triple eau dans huile dans eau,
- une émulsion triple huile dans eau dans huile,
- une émulsion huile dans eau contenant des cristaux liquides,
- des émulsions complexes contenant des cristaux liquides formant des bicouches lipidiques entourant des phases grasses,
- des pseudo-émulsions (dispersion d'une phase huileuse ou d'une émulsion eau dans huile dans une phase aqueuse gélifiée, sans tensio-actifs traditionnels,
- des micro-émulsions huile dans eau ou eau dans huile,
- des émulsions contenant deux phases huiles dispersées, différentes et insolubles entre elles,
- une pseudo émulsion ou dispersion d'une phase grasse dispersée dans une phase aqueuse et stabilisée avec du Lubragel®, polyglycérol méthacrylate propylène glycol du Pemulen®, des polymères type alkyl arylate trans polymère, Hypan®, de la gomme xanthane, de la CMC, de l'hydroxyethyl cellulose, des polysaccharides type Amigel®, de la Polyvinylpyrrolidone, ou un mélange de deux ou plusieurs de ces gélifiants.

Il est évident que les micro-capsules contenant des filtres solaires et durcies convenablement sont insolubles dans les diverses phases des compositions citées et constituent donc une phase supplémentaire.

Les compositions cosmétiques peuvent contenir au moins 1 à 50 % des micro-capsules contenant des filtres solaires. Des micro-capsules contenant des filtres solaires différents peuvent être ajoutées dans une même composition.

Enfin des réflecteurs solaires peuvent être ajoutés à ces compositions cosmétiques pour en renforcer le pouvoir protecteur.

Ce sont généralement des produits insolubles dans les phases huileuses et aqueuses et constituent donc une phase supplémentaire. Ils seront choisis par exemple parmi les produits suivants :
- les perfluoroéthers comme les FOMBLIN® de la Sté Montecotini,
- les pigments insolubles comme :
   - les oxydes de titane
   - Oxyde de titane rutile
   - Oxyde de titane anatase
   - Oxyde de titane pyrogéné comme le P 25® de Degussa
   - Oxyde de titane micronisé dans le SUN VEIL® d'Ikeda
   - Oxyde de titane traité en surface par des silicones ou par des acides aminés, ou par de la lécithine, ou par des stéarates métalliques,
   - Oxyde de fer
   - Oxyde de fer traité en surface par des silicones, ou par des acides aminés, ou par de la lécithine, ou par des stéarates métalliques,
   - Oxyde de zinc
   - Oxyde de zinc micronisé comme l'UFZO® de cosmo trends corporation
   - Mica recouvert d'oxyde de titane.

La présente invention a également pour objet l'application à titre de produit cosmétique des compositions telles que définies ci-dessus.

La présente invention a aussi pour objet une méthode de traitement cosmétique caractérisée en ce que l'on utilise les compositions cosmétiques telles que définies ci-dessus. Dans la méthode de traitement cosmétique utilisant les compositions de l'invention, lesdites compositions sont utilisées par application cutanée comme les compositions usuelles.

La présente invention a aussi pour objet à titre de médicament cosmétique, les microcapsules telles que définies ci-dessus.

La présente invention a particulièrement pour objet les compositions pharmaceutiques caractérisées en ce qu'elles comprennent des microcapsules telles que définies ci-dessus.

De manière générale, les applications thérapeutiques des compositions pharmaceutiques de l'invention peuvent être les mêmes que celles décrites ci-dessus pour les compositions cosmétiques et notamment la prévention et le traitement des affections cutanées dues aux rayonnements UVA et UVB.

Les compositions pharmaceutiques comprenant les microcapsules selon l'invention peuvent être préparées de manière similaire à la préparation des compositions cosmétiques décrites ci-dessus.

Les exemples suivants illustrent l'invention sans toutefois la limiter.

### Exemple 1 : Exemple de préparation de micro-capsules dures contenant du PARSOL MCX® (octyl méthoxycinnamate)

10 g de gélatine sont mis dans 120 ml d'eau déminéralisée à 80°C et on disperse ensuite 90 ml de Parsol MCX®.

La coacervation est produite par l'ajout à 55°C environ, d'une solution de 1 g d'hexamétaphosphate de sodium dans 20 ml d'eau distillée. On dilue l'ensemble avec 100 ml d'eau distillée.

Ensuite on diminue le pH par addition d'acide acétique glacial jusqu'à un pH de 4,2 environ. Puis le milieu réactionnel est refroidi brutalement jusqu'à 5°C et il est maintenu environ 30 minutes à cette température.

Le durcissement par réticulation est réalisée par l'addition de 5 ml d'une solution de glutaraldéhyde à 25 %. On laisse agir pendant 12 à 24 h à température ambiante. Ensuite les microcapsules sont isolées par filtration et séchées par exemple dans un lit d'air fluidisé.

### Exemple 2 : Exemple de préparation de micro-capsules dures contenant du PARSOL MCX®

On disperse dans la solution de 10 g de gélatine dans 120 ml d'eau déminéralisée à 80°C, 90 ml de PARSOL MCX® contenant de 0,5 à 5 % de polysorbate 20, tensio actif non ionique destiné à augmenter le rendement en micro-capsules et à obtenir une taille plus homogène de micro-capsules. On obtient ainsi une population dont la taille va de 30 à 80 micromètres, avec un maximum (plus de 30 %) entre 40 et 45 micromètres.

### EXEMPLES DE COMPOSITIONS

### Exemple 3 : (composition comparative)

On prépare une émulsion huile dans eau de la manière suivante :
On chauffe à 80°C les composants de la phase grasse suivante :

| | |
|---|---|
| - Stéarate de glycérol autoémulsionable (arlacel 165® de la Sté ICI) | 6 g |
| - Alcool cétylique | 1 g |
| - stérol de soja éthoxylé (générol 122 E 10® de la Sté Henkel) | 2 g |
| - mélange d'huile de vaseline et d'alcool de lanoline (amerchol L101 de la Sté Amerchol®) | 3 g |
| - Petrolatum et alcool de lanoline (Amerchol CAB® de la Sté Amerchol) | 1 g |
| - Huile de carthame | 6 g |
| - Beurre de Karité | 3 g |
| - Propyl paraben | 0,05 g |

Par ailleurs on prépare la phase aqueuse suivante que l'on porte également à 80°C :

| | |
|---|---|
| - eau déminéralisée | 60 g |
| - Sorbitol à 70 % | 3 g |
| - Gomme xanthane | 0,3 g |
| - Methyl paraben | 0,1 g |

Quand la gomme xanthane est bien dispersée, on ajoute la phase grasse à la phase aqueuse à 80°C, et on agite vivement pendant 20 minutes. L'émulsion se forme.

Ensuite on réduit l'agitation et on refroidit lentement l'émulsion jusqu'à 40°C.

On y ajoute alors 2 g d'eau contenant 0,15 g d'imidazolidinyl urée, puis 0,3 g de parfum.

A cette température on ajoute 6,6 g de microcapsules préparées comme indiqué ci-dessus à l'exemple 1 ou 2 de la présente invention.

Ces capsules contiennent 80 % de Parsol MCX® pur par rapport au poids de microcapsules.

La préparation contient donc 5,28 % de Parsol MCX® pur.

### Exemple 4 :

La même préparation que celle décrite dans l'exemple 3, est additionnée de 9,9 % de microcapsules, ce qui représente un dosage à 7,92 % de Parsol MCX® pur.

### Exemple 5 : (composition comparative)

a) On prépare un gel gras suivant la formule :

| | |
|---|---|
| - Huile de vaseline épaisse | QS 100 g |
| - Stéarate d'octyle | 10,0 g |
| - Beurre de karité | 2,0 g |
| - Esters d'acide glycol C₁₈ - C₃₆ (synchrowax de la Sté CRODA) | 4,0 g |
| - Huile d'Onagre | 1,0 g |
| - Huile de carthame | 5,0 g |
| - Propyl paraben | 0,1 g |

Ces produits sont chauffés jusqu'à homogénéité. On ajoute ensuite 3 g de silice pyrogénée (aérosil 200 de la Sté DEGUSSA) sous bonne agitation.

Quand la silice est bien dispersée, on ajoute 3 % d'une solution d'extrait de silymarine dans le polyéthylène glycol 400 ; 0,2 % de N-acétyl tyrosine et 0,3 % d'une composition parfumante.

On ajoute ensuite 10 g de micro-capsules préparées en laboratoire suivant la méthode décrite ci-dessus et contenant du Parsol MCX® pur.

La teneur en Parsol MCX® est de 76 % du poids total des micro-capsules.

On voit donc que la composition contient 7,6 % de Parsol MCX®.

### Test de détermination du facteur de protection solaire des microcapsules de filtre solaire

### Source UV :

La source de lumière UV est une lampe Xénon de 150 watts, sans ozone, filtrée avec WG 320 de 1 mm. Après réglage des flux par un diaphragme, la lumière est dirigée sur 6 guides liquides qui délivrent une lumière froide. L'extrémité des guides est située à un millimètre de la peau du dos à tester. Les guides sont maintenus assemblés par un bloc permettant leur application simultanée.

### Dosimétrie :

Un dosimètre Robertson-Berger calibré, pour la réponse de l'épiderme humain aux UV solaires, est utilisé pour calculer les doses délivrées à l'extrémité de chaque guide. Les doses sont ajustées à l'aide des diaphragmes et sont en progression de 1,25. Le dosimètre exprime les doses en DEM par minute. Les UV sont délivrés pendant le même temps pour chaque guide.

### Examen clinique préalable :

Chaque volontaire a eu un examen physique de la zone de test, le dos entre la ceinture et le sommet des omoplates, afin de déterminer l'éventuelle présence de :
- érythème solaire, pigmentation résiduelle, cicatrice, lésions épidermiques et dermiques diverses, et anomalies de pigmentation de la peau.

### Volontaires :

7 volontaires humains en bonne santé sans anamnèse de photosensibilité, n'ayant pas été exposés au soleil depuis 3 mois, ont été sélectionnés parmi l'ensemble des volontaires. Chacun d'eux a été informé des buts, procédés et risques potentiels de ce test.

Un consentement informé a été obtenu pour chaque volontaire avant le début du test.

### Typage de la peau des sujets :

Avant les expositions, chaque volontaire répond à un questionnaire écrit, en 5 points, correspondant à des situations différentes de sensibilité solaire ou de capacité à acquérir un hâle. En fonction des réponses, les sujets sont placés dans une des 6 catégories représentant les mélanogénotypes des peaux caucasoïdes, et en 4 phototypes.

### Détermination préalable de la Dose Minimale Erythémale :

Afin de déterminer la sensibilité UV des volontaires, une série de 6 expositions UV a été appliquée dans la zone de traitement, 24 heures avant le test, chaque exposition étant 1,25 fois plus importante que l'exposition précédente.

24 heures après l'irradiation, les zones irradiées ont été examinées et la dose minimale érythémale (DEM) est enregistrée. Cette DEM est alors utilisée comme un indicateur pour fixer le temps d'exposition nécessaire au test principal.

### Détermination de la zone de test :

Sur le dos, sont tracées les limites de gabarit d'une surface de 100 cm**²**. A chaque zone correspond un produit appliqué.

### Application du produit à tester et du standard :

Le produit à tester a été pesé par unités de 2,2 g et le standard a été introduit dans une seringue, type insuline, sans bulles d'air. Une quantité de 2 mg/cm**²**, soit 0,2 ml, est appliquée sur les zones tests (100 cm**²**). Les produits sont étalés au doigtier très uniformément par massage léger de quelques minutes.

### Période d'attente :

Après application, une période de repos de 15 minutes est respectée avant l'irradiation.

### Exposition de la zone test :

L'appareil portant les guides est appliqué contre la zone de test, et l'irradiation est poursuivie pendant le temps proportionnel à la DEM et au PF (facteur de protection) théorique du produit.

Après l'irradiation, chaque volontaire est instruit de ne pas s'exposer à des radiations UV et de venir pour examens 24 heures après.

### Détermination de la réponse :

24 heures ± 2 heures après l'irradiation, la réponse de la peau traitée est évaluée selon les critères suivants :
- absence d'érythème
- trace d'érythème
- érythème incomplet ou irrégulier
- érythème régulier bien défini - DEM
- érythème important
- érythème violacé avec oedème (dose minimale oedémateuse = 3 DEM).

### Calcul du facteur de protection solaire :

Le facteur de protection solaire individuel pour chaque volontaire est le rapport des valeurs de la DEM en peau traitée et non traitée. Les résultats individuels (20) sont additionnés, et la moyenne arithmétique est calculée.

On obtient les résultats suivants :

| SUPPORT | TENEUR EN PARSOL MCX | TAILLE DES MICRO-CAPSULES | INDICE DE PROTECTION UVB |
|---|---|---|---|
| EMULSIONS | | | |
| Ex 3 | 5.28 | 100-125 | 5,16 |
| Ex 4 | 7.92 | | 6,95 |
| | 6.6 | pas de micro-capsule | 4,37 |

| GEL GRAS | | | |
|---|---|---|---|
| Ex 5 | 7.6 | 100-125 | 10.3 |
| | 7.6 | pas de micro-capsule | 9.3 |

Ces résultats montrent que l'indice de protection des filtres solaires placés dans les micro-capsules étanches est supérieur à celui des filtres solaires non encapsulés.

## Revendications

1. Microcapsules comprenant un ou plusieurs filtres solaires caractérisées en ce que lesdites microcapsules sont durcies de façon à être étanches et que la taille des microcapsules est comprise entre 30 et 80 micromètres.

2. Microcapsules telles que définies à la revendication 1 caractérisées en ce que la paroi des microcapsules comprend de la gélatine durcie de préférence par du glutaraldéhyde.

3. Microcapsules telles que définies aux revendications 1 à 2 caractérisées en ce que le ou les filtres solaires qu'elles comprennent sont purs ou dilués dans une huile et choisis notamment dans la liste suivante : octyl méthoxycinnamate et notamment le 4-méthoxycinnamate d'éthyl hexyle, méthylbenzylidène camphre, isoamyl méthoxycinnamate, octydiméthyl paba, paba, homosalate, octyl salicylate, butyl méthoxydibenzoylméthane, isopropyl dibenzoylméthane, oxybenzone, octyl triazone.

4. Procédé de préparation de microcapsules telles que définies aux revendications 1 à 3 comprenant une préparation de solution colloïdale et une dispersion de la substance à encapsuler dans cette solution, une séparation de phase (ou coacervation) et formation d'un système ternaire par variation du pH et une encapsulation de la substance dispersée, caractérisé en ce que ce procédé comprend ensuite une phase de durcissement de la paroi desdites microcapsules et séparation desdites micro-capsules afin d'obtenir des particules solides.

5. Procédé tel que défini à la revendication 4 caractérisé en ce que l'on ajoute un tensio actif non ionique tel que le MONTANE 20 dans la dispersion de la substance à encapsuler.

6. Compositions cosmétiques caractérisées en ce qu'elles comprennent des microcapsules telles que définies à la revendication 1.

7. Application à titre de produit cosmétique des compositions telles que définies à la revendication 6.

8. Méthode de traitement cosmétique caractérisée en ce que l'on utilise les compositions cosmétiques telles que définies à la revendication 6.

9. A titre de médicament cosmétique, les microcapsules telles que définies à la revendication 1.

10. Compositions pharmaceutiques caractérisées en ce qu'elles comprennent des microcapsules telles que définies à la revendication 1.

## Claims

1. Microcapsules containing one or more sun filters characterized in that said microcapsules are hardened so as to be impervious and that the size of the microcapsules is comprised between 30 and 80 micrometers.

2. Microcapsules as defined in claim 1 characterized in that the wall of the microcapsules contains gelatin preferably hardened by glutaraldehyde.

3. Microcapsules as defined in claims 1 to 2, characterized in that the sun filter or filters that they contain are pure or diluted in an oil and chosen notably from the following list: octyl methoxycinnamate and notably ethyl hexyl 4-methoxycinnamate, camphorous methylbenzylidene, isoamyl methoxycinnamate, octydimethyl paba, paba, homosalate, octyl salicylate, butyl methoxydibenzoylmethane, isopropyl dibenzoylmethane, oxybenzone, octyl triazone.

4. Preparation process for microcapsules as defined in claims 1 to 3 containing a preparation of colloidal solution and a dispersion of the substance to be encapsulated in this solution, a phase separation (or coacervation) and formation of a ternary system by variation of the pH and an encapsulation of the dispersed substance, characterized in that this process then contains a hardening phase of the wall of said microcapsules and separation of said microcapsules in order to obtain solid particles.

5. Process as defined in claim 4 characterized in that a non-ionic surfactant such as MONTANE 20 is added to the dispersion of the substance to be encapsulated.

6. Cosmetic compositions characterized in that they contain the microcapsules as defined in claim 1.

7. Use as cosmetic product of the compositions as defined in claim 6.

8. Cosmetic treatment method characterized in that the cosmetic compositions as defined in claim 6 are used.

9. As cosmetic medicament, the microcapsules as defined in claim 1.

10. Pharmaceutical compositions characterized in that they contain the microcapsules as defined in claim 1.

## Patentansprüche

1. Mikrokapseln mit einem oder mehreren Sonnenschutzmitteln, dadurch gekennzeichnet, daß besagte Mikrokapseln gehärtet sind, so daß sie dicht sind, und daß die Größe der Mikrokapsein zwischen 30 und 80 Mikrometern liegt.

2. Mikrokapseln wie in Anspruch 1 definiert, dadurch gekennzeichnet, daß die Wand der Mikrokapseln Gelatine enthält, die vorzugsweise mit Glutaraldehyd gehärtet ist.

3. Mikrokapseln wie in den Ansprüchen 1 bis 2 definiert, dadurch gekennzeichnet, daß das oder die Sonnenschutzmittel, die sie enthalten, rein oder mit einem Öl verdünnt sind und insbesondere aus der folgenden Liste ausgewählt sind: Octylmethoxycinnamat und insbesondere Ethylhexyl-4-methoxycinnamat, Methylbenzylidencampher, Isoamylmethoxycinnamat, Octyldimethyl-PABA, PABA, Homosalat, Octylsalicylat, Butylmethoxydibenzoylmethan, Isopropyldibenzoylmethan, Oxybenzon, Octyltriazon.

4. Verfahren zur Herstellung von Mikrokapslen wie in den Ansprüchen 1 bis 3 definiert, das eine Herstellung einer kolloidalen Lösung beinhaltet und eine Dispersion der in dieser Lösung zu verkapselnden Substanz, eine Phasentrennung (oder Koazervation) und die Bildung eines ternären Systems durch Veränderung des pH und eine Verkapselung der dispergierten Substanz, dadurch gekennzeichnet, daß dieses Verfahren dann eine Härtungsphase der Wand besagter Mikrokapseln beinhaltet und die Abtrennung besagter Mikrokapseln, um feste Teilchen zu erhalten.

5. Verfahren wie in Anspruch 4 definiert, dadurch gekennzeichnet, daß man in die Dispersion der zu verkapselnden Substanz ein nicht ionisches Tensid wie MONTANE 20 zugibt.

6. Kosmetische Zusammensetzungen, dadurch gekennzeichnet, daß sie Mikrokapseln enthalten, sie wie in Anspruch 1 definiert sind.

7. Anwendung der Zusammensetzungen wie in Anspruch 6 definiert als kosmetisches Mittel.

8. Kosmetische Behandlungsmethode, dadurch gekennzeichnet, daß man die kosmetischen Zusammensetzungen verwendet, wie sie in Anspruch 6 definiert sind.

9. Die Mikrokapseln wie in Anspruch 1 definiert als kosmetisches Arzneimittel.

10. Pharmazeutische Zusammensetzungen, dadurch gekennzeichnet, daß sie Mikrokapseln enthalten, wie sie in Anspruch 1 definiert sind.
